# EUROPEAN PATENT APPLICATION

(11) **EP 3 778 875 A1**
(43) Date of publication of application: **17.02.2021**
(21) Application number: 19191867.1
(22) Date of filing: 14.08.2019
(51) Int. Cl.: C12N 5/077

(54) **IMMORTALIZED MYOBLAST CELL LINES AND USES THEREOF**

(71) Applicant: MaxiVax SA, 1206 Geneve (CH); Les Hôpitaux Universitaires de Genève, 1205 Geneva (CH); UNIVERSITE DE GENEVE, 1211 Geneva 4 (CH)
(72) Inventor: LATHUILIÈRE, Aurélien, 1202 Geneva (CH); MACH, Nicolas, 1245 Collonge-Bellerive (CH); SALMON, Patrick, 1218 Le Grand-Saconnex (CH)
(74) Representative: reuteler & cie SA

(57) **Abstract**

The present invention relates to immortalized human cells particularly useful for cell encapsulation therapy and methods of preparation and use thereof.

## Description

### Field of the Invention

The present invention relates generally to the field of encapsulation and in particular the development of immortalized cell lines and methods for producing the same for use as encapsulated cellular implants for the release of therapeutic proteins or adjuvants.

### Background of the Invention

Cell encapsulation technology allows for chronic and/or localized administration of macromolecules in multiple domains. It is based on the implantation in a subject of one or several biocompatible capsules containing genetically modified cells to produce a therapeutic protein of interest. This type of capsule is generally made up of a semi-permeable membrane that provides a mechanical protection and isolates the modified cells, thus avoiding the contact between the transplanted cells and the immune cells of host and resulting in prolonged survival of the encapsulated cells. In addition, the semipermeable membrane allows the influx of nutrients and oxygen to the transplanted cells and the efflux of the protein of interest to the host, thus allowing a continuous and prolonged production.
*Ex vivo* gene therapy using retrievable encapsulated cellular implants has been developed as an effective strategy for the local and/or chronic delivery of therapeutic proteins. In particular, modulating the activity of the immune system of a patient has been considered as an innovative approach for the treatment of various disorders and in particular, therapeutic schemes using genetically engineered encapsulated cells have been developed such as chronic administration of monoclonal antibodies for passive immunization against neurodegenerative diseases and the local delivery of a cytokine as an adjuvant for anti-cancer vaccines (Lathuilière et al., 2015, Int. J. Mol. Sci., 16, 10578-10600).

It has been recently developed a technique for anti-cancer active immunization using encapsulated cells secreting granulocyte-macrophage colony stimulating-factor (GM-CSF) allowing the standardized release of GM-CSF by the genetically modified allogeneic cells (MVX-1 cells) which has immune-protective and boosting activities useful for tumor regression. Patient immunizations is performed in healthy skin, distant from tumor deposits by combining irradiated autologous tumor cells and 2 capsules containing MVX-1 cells, producing >20ng/24h of huGM-CSF. This permits the production of GM-CSF and exposes the immune system to the tumor-associated antigens (TAA) expressed by the autologous tumor cells at the injection site. Local expression of GM-CSF recruits and activates antigen-presenting cells (APC), which induces both antibody-dependent cell-mediated cytotoxicity (ADCC) and cytotoxic T-lymphocyte responses at the site of the injection and systemically (WO 2017/064571) *as presented in* Mach et al., 2015, Annals of Oncology,26 (suppl_8): 1-4. 10.1093/annonc/mdv513*.*
In order to optimize the human application of encapsulation technologies, a critical point is the generation of an implantable, safe and usable human cell lines as a platform for the *in vivo* secretion of recombinant proteins of interest. Therefore, there is a need for the development of newly developed efficient cell lines particularly suitable encapsulation technology in terms of safety and long-lasting effects.

### Summary of the Invention

The present invention is based on the unexpected findings of a method for the preparation and selection of immortalized human myoblasts allowing achieving immortalized human myoblasts with exceptional survival characteristics in cell encapsulation devices, in particular able to differentiate under difficult metabolic conditions, while maintaining high secretion level of recombinant protein.

In particular, the method of immortalization of human myoblasts according to the invention comprises the transduction of human myoblasts isolated from a muscle tissue from a donor (e.g. healthy or autologuous) with at least one lentiviral vector (such as pCLX type or pRRLSIN or other suitable lentiviral vectors such as those as described in Giry-Laterrière, 2011, Methods Mol Biol., 737:183-209) encoding for CDK4 protein and at least one lentiviral vector (such as pCLX type or pRRLSIN or other suitable lentiviral vectors such as those as described in *Giry-Laterrière, 2011, supra*) encoding for hTERT protein and the selection of at least one immortalized individual clone for its improved in vitro proliferation and stability properties such as improved survival when encapsulated compared to parent cell line using single cell cloning techniques. In particular, a selected immortalized human myoblast cell line obtained by a method of the invention such as for example the cell line deposited under number CCOS 1902 advantageously presents long term survival and proliferation ability in a biocompatible capsule and long term keeping myoblast features.

Further, another aspect of the invention is based on the unexpected finding that immortalized human myoblast cell lines of the invention can be easily genetically engineered to express proteins of interest, particularly useful in the field of encapsulation therapy technologies and it is provided a method for the preparation of genetically modified immortalized human myoblasts, wherein said method comprises the transduction of immortalized human myoblasts according to the invention with at least one lentiviral vector (e.g. pCLX Or pRRLSIN type) encoding for the protein of interest under the control of a promoter which is overactivated in hypoxia condition such as PGK (phosphoglycerate kinase) promoter. Under the control of this promoter, protein secretion by the genetically modified immortalized human myoblasts according to the invention is advantageously increased by a factor of 2 to 3 under hypoxia conditions which makes these cells even more interesting for use in cell encapsulation technology which subjects cells to hypoxia. In particular, a genetically modified immortalized human myoblast cell line secreting GM-CSF obtained by a method of the invention such as for example the cell line deposited under number CCOS 1901 advantageously presents long term survival and proliferation ability in an implantable device and long term keeping myoblast features with high levels of secretion of GM-CSF which are highly useful for cancer cell therapy.

An object of this invention is to provide an immortalized human cell line useful for cell encapsulation technology.

It is useful to provide an immortalized human cell line which is not tumorogenic and present a prolonged survival even in hypoxic conditions.

It is useful to provide an immortalized human cell line which is able to be genetically engineered to secrete high levels of protein interest.

It is useful to provide genetically engineered immortalized human cells which are able to maintain high levels of secretion of a proteins interest in an encapsulation device.

It is useful to provide genetically engineered immortalized human cells which are able to be frozen and thawed after being loaded in an encapsulation device and maintain high levels of secretion of a proteins interest.

Objects of this invention have been achieved by providing an immortalized human myoblast cell line according to claims 3 and 5, a method for obtaining an immortalized human myoblast cell line according to claim 1 and uses thereof.

Objects of this invention have also been achieved by providing genetically engineered immortalized human myoblast cells according to claims 9 and 10, a method for obtaining genetically engineered immortalized human myoblast cells according to claim 7 and uses thereof.

Disclosed herein, according to a first aspect of the invention, is a method of establishing an immortalized human myoblast cell line, said method comprising the steps of:
a) Transducing at least one primary human myoblast cell with a lentiviral vector encoding for the cyclin-dependent kinase 4 (CDK4) gene and a lentiviral vector encoding for the catalytic subunit of the human telomerase (hTERT) gene to obtain immortalization of the said primary human myoblast cell;
b) Growing cells from at least one immortalized primary human myoblast cell obtained under step a) in a myoblast cell growth medium and isolating from the growth medium each obtained cell presenting at least one myoblast phenotypic marker in a separate culture medium;
c) Growing separately in individual culture and amplification media each isolated cell obtained under step b);
d) Selecting among all the individual culture and amplification media of step c) at least one cell line with improved stability or expression properties from the parental cell by single cell cloning;
e) Controlling the myogenic potential of the selected at least one cell line;
f) selecting individual clones based on their ability to survive in an encapsulation device;
g) optionally repeating steps c) to f) sequentially to further improve the selected line.

According to another aspect of the invention, is provided an immortalized human myoblast cell line or a composition comprising immortalized human myoblast cells or a progeny thereof derived from primary human myoblast cells, wherein said cells express CDK4 and hTERT and retain myoblast features.

According to another aspect of the invention, is provided an immortalized human myoblast cell line deposited with CCOS under accession number 1902 or a composition or a progeny thereof.

According to another aspect of the invention, is provided an immortalized human myoblast cell line or a composition comprising immortalized human myoblast cells or a progeny thereof derived from primary human myoblast cells obtainable from a method according to the invention.

According to another aspect of the invention, is provided a method for the preparation of genetically engineered immortalized human myoblast cells expressing a therapeutic protein under hypoxic conditions.

According to another aspect of the invention, is provided a genetically engineered immortalized human myoblast cell line according to the invention or a composition or a progeny thereof.

According to another aspect of the invention, is provided a genetically immortalized human myoblast cell line secreting GM-CSF deposited with CCOS under accession number 1901 or a composition or a progeny thereof.

According to another aspect of the invention, is provided a genetically engineered immortalized human myoblast cell line or a composition or a progeny thereof obtainable from a method according to the invention.

According to another aspect of the invention, is provided genetically engineered immortalized human myoblast cells of the invention for use in encapsulated cell therapy.

According to another aspect of the invention, is provided a pharmaceutical composition comprising at least one genetically engineered immortalized human myoblast cell according to the invention and a pharmaceutically acceptable carrier, diluent or excipient thereof.

According to another aspect, the invention provides genetically engineered immortalized human myoblast cells of the invention for use in the prevention and/or treatment of a disorder or a disease, in particular a cancer, an inflammatory disorder or a neurodegenerative disorder.

According to another, the invention provides a use of genetically engineered immortalized human myoblast cells of the invention for the preparation of a pharmaceutical composition or of an implantable encapsulated cell device for the prevention and/or treatment of a disorder or a disease, in particular a cancer, an inflammatory disorder or a neurodegenerative disorder.

According to another, the invention provides an implantable biocompatible device or a kit comprising at least one immortalized human myoblast cell according to the invention in a cell culture medium.

According to another aspect, the invention provides a method of preventing or treating a disorder or a disease associated in a subject, in particular a cancer, an inflammatory disorder or a neurodegenerative disorder, said method comprising administering in a subject in need thereof a therapeutically effective amount of genetically engineered immortalized human myoblast cells of the invention.

### Description of the figures

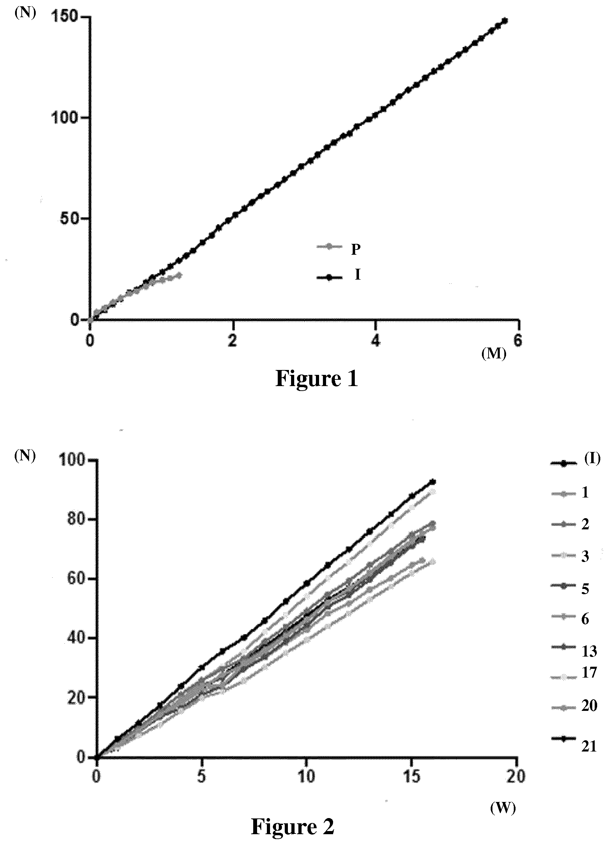

**Figure 1** represents the proliferation ability expressed as the number of cell population double (N) versus the number of months (M) after transduction of immortalized human myoblast of the invention (I), i.e. before step d) of a process of the invention compared to primary myoblast cells (P), as measured by cell counting at each passage as described in Example 1.
**Figure 2** represents the proliferation ability expressed as the number of cell population double (N) versus the number of weeks (W) after sorting of the clones from the immortalized human myoblast population (I) of the 9 selected clones as described in Example 1.

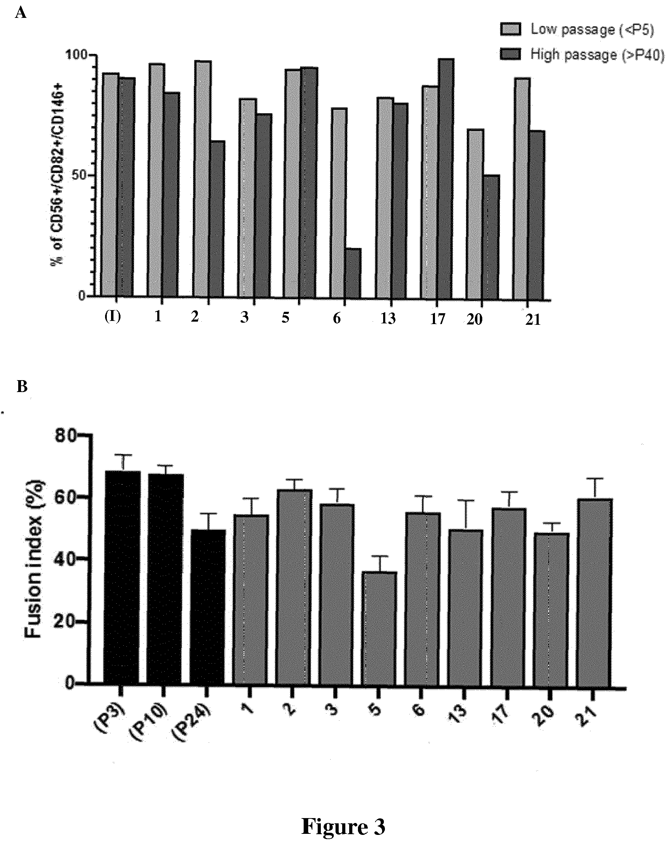

**Figure 3** shows the preserved myogenic characteristics of the clones obtained by a method of the invention after immortalization and sorting (after step d)) compared to the immortalized population as measured by the maintenance of their ability to express myogenic markers over time quantified by flow cytometry compared to immortalized cell population before sorting (I) **(A)** and their preserved capacity to fuse and differentiate into myotubes was quantified by immunohistochemistry **(B)** compared to primary cells (P3) and immortalized cell populations (P10) and (P24) after 10 and 24 passages, respectively as described in Example 1.

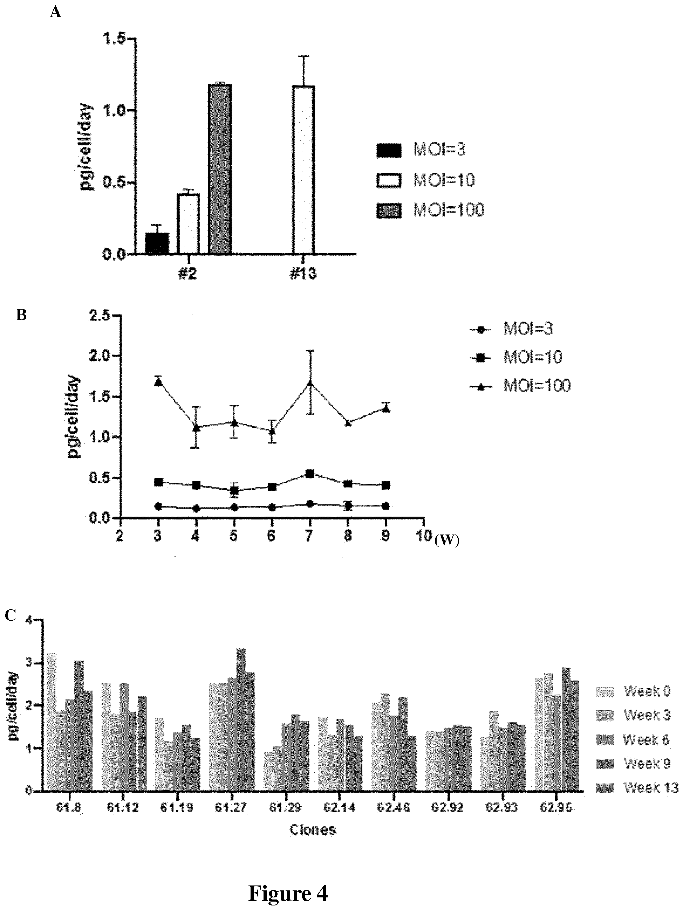

**Figure 4** shows GM-CSF secretion rate as measured by ELISA achieved by genetically modified immortalized human myoblast cells of the invention (clone 2) right after transduction **(A)**, versus the number of weeks (W) after transduction for clone 2 **(B)** and for various clones after sorting step (iv) of the method for different weeks after transduction **(C)**, as described in Example 3.

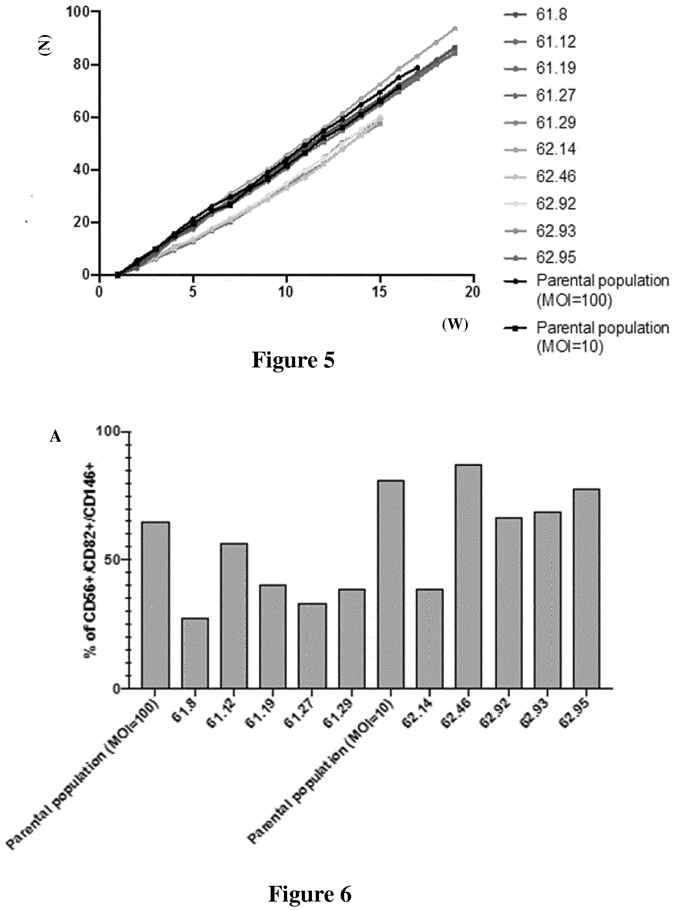

**Figure 5** shows the proliferation ability expressed as the number of cell population double (N) versus the number of weeks (W) after sorting of the clones from the genetically modified immortalized human myoblast population of the 10 selected clones as described in Example 3.

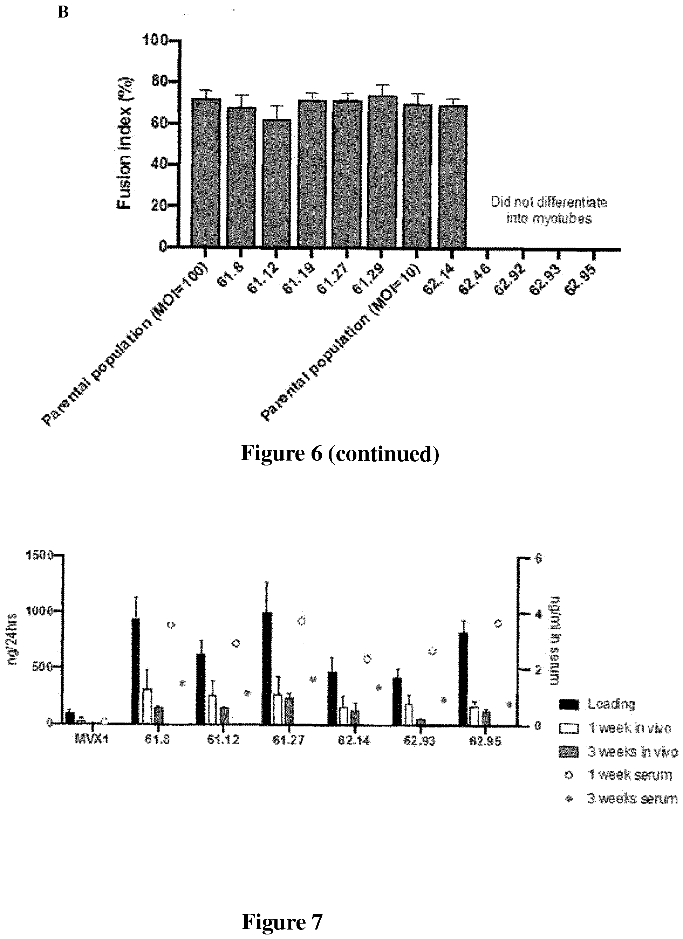

**Figure 6** shows the preserved myogenic characteristics of the clones obtained by a method of the invention after sorting (after step iv)) as measured by the maintenance of their ability to express myogenic markers over time quantified by flow cytometry **(A)** and their preserved capacity to fuse and differentiate into myotubes was quantified by immunohistochemistry **(B)** compared to the parental populations (MOI=10 and 100) as described in Example 2.
**Figure 7** shows the GM-CSF secretion rate as measured by ELISA achieved by various encapsulated selected clones for different weeks after *in vivo* transplantation as compared to control encapsulated cells (MVX-1), as described in Example 3.

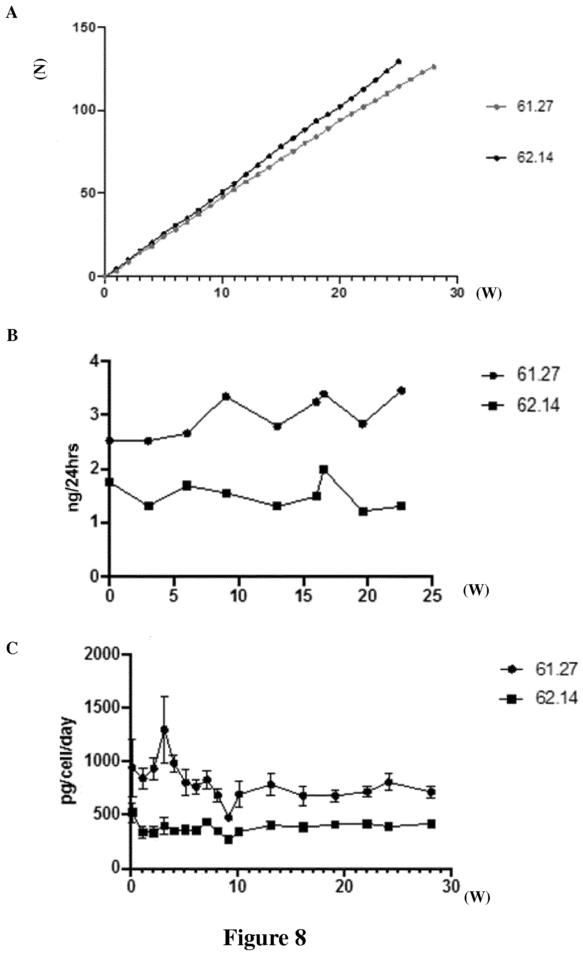

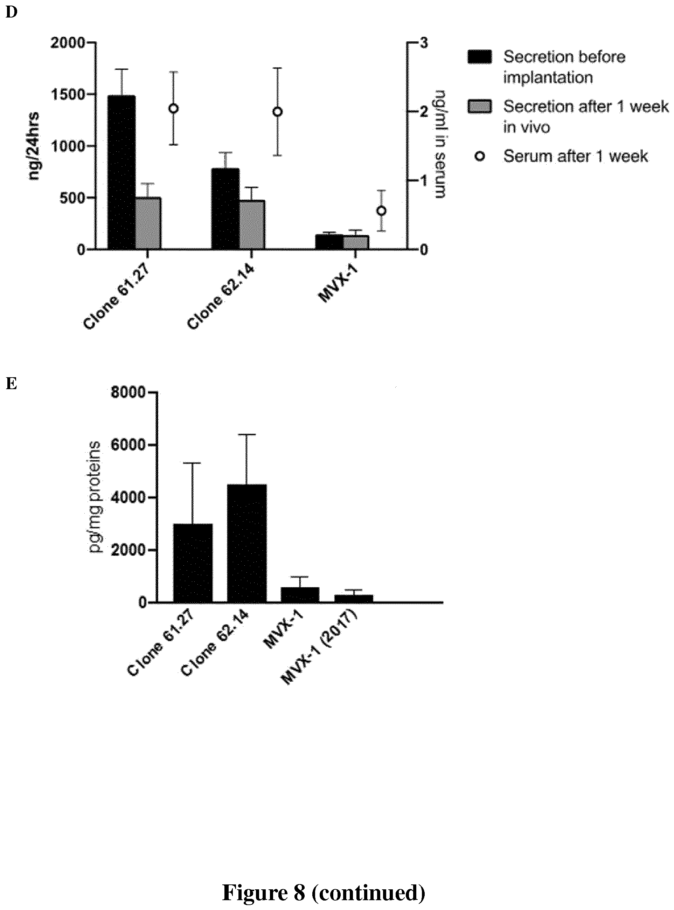

**Figure 8** shows the stability over time of two selected clones 61.27 and 62.14 in terms of *in vitro* proliferation rate as non-encapsulated cells **(A)** expressed as the number of cell population double (N) versus the number of weeks (W) after sorting of the clones from the genetically modified immortalized human myoblast population, *in vitro* secretion rate as non-encapsulated cells versus weeks (W) after sorting **(B)** and *in vitro* secretion rate as encapsulated cells versus weeks (W) after encaspulation **(C)** and *in vivo* secretion rate in mice as encapsulated cells measured by quantification of GM-CSF in serum compared before implantation **(D)** and in tissue surrounding the capsule **(E)**, compared to control encapsulated cells (MVX-1), as described in Example 3.

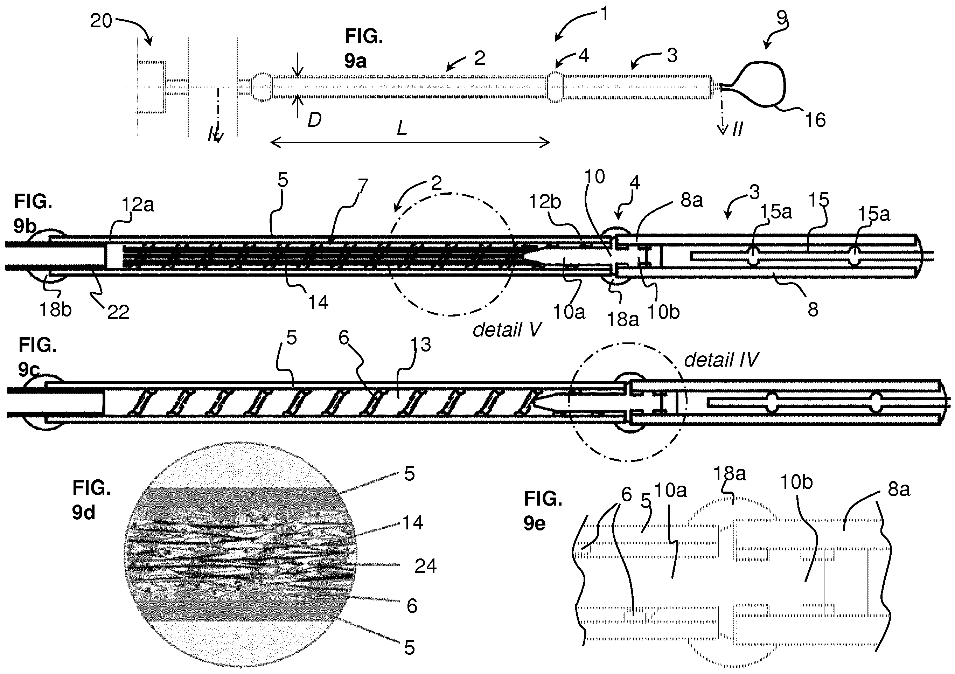

**Figure 9** is a representation of an implantable encapsulation device used in the experiments with encapsulated cells of the invention, **a:** is a schematic illustration of an implantable capsule according to an embodiment of this invention. **b**: is a cross-sectional view through line II-II of **figure 9a**; **c**: is a cross-sectional view similar to **figure 9b** with an internal matrix of the capsule removed; **d**: is a detailed view of circle IV of **figure 9c** of the cell receiving portion 2 inside of the implantable encapsulation device containing the immortalized myoblasts 24 according to the invention. **e**: is a detailed view of circle V of **figure 9b**.

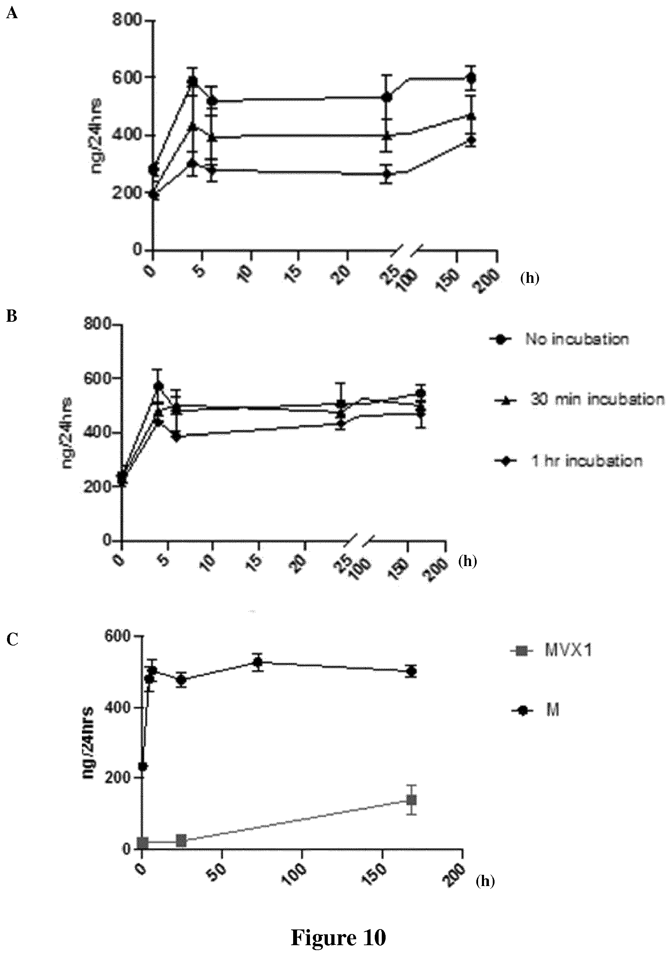

**Figure 10** shows GM-CSF secretion rate as measured by ELISA achieved by frozen encapsulated genetically modified immortalized human myoblast cells (M) of the invention versus time after thawing (in hours) formulated in 5% **(A)** and 10 % **(B and C)** glycerol as freezing medium with different time of incubation with the freezing medium and compared to control encapsulated cells (MVX-1), as described in Example 3.

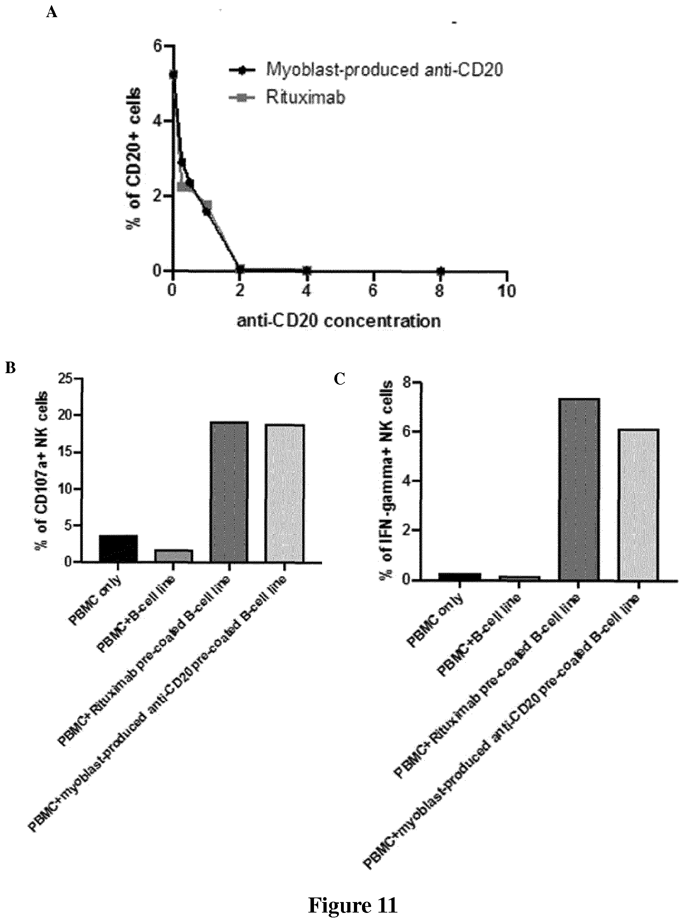

**Figure 11** supports the ability of immortalized myoblast cells obtained under Example 1 from clone 2 transduced with lentiviral vectors encoding for the heavy and light chain of rituximab to produce biologically active monoclonal antibody as described in Example 4. **A:** IgG secreted by genetically modified cells of the invention and commercial Rituximab behave identically when incubated with a fluorescent tagged anti-CD20 antibody; **B & C:** myoblast-produced IgG was as efficient in a degranulation assay that tested the induction of a cytotoxic response (overexpression of IFN-gamma (B) or CD107a(C)) after a B-cell line was exposed to either Rituximab or myoblast-produced anti-CD20.

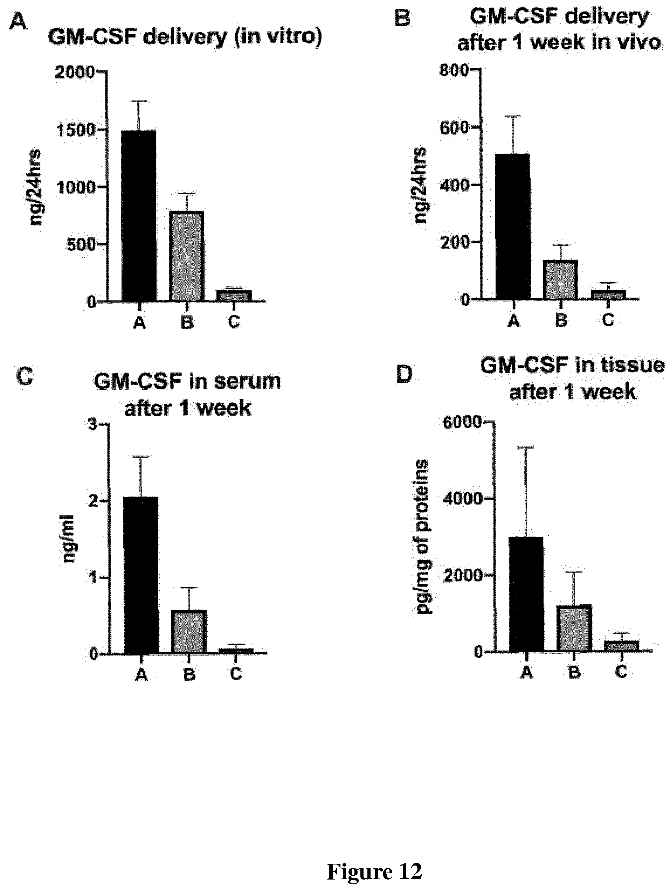

**Figure 12** compares the release of huGM-CSF from two distinct capsules and two types of cells as described in Example 5. **A:** GM-CSF release *in vitro* in the capsule supernatant for **Group A:** a capsule according to an embodiment of the invention (illustrated in Figure 9) containing genetically modified immortalized human myoblast cells secreting the same therapeutic protein GM-CSF, **Group B**: a control cell line being K562 human erythroleukemia cells expressing human GM-CSF loaded in the same capsule according to an embodiment of the invention (illustrated in Figure 9) and **Group C:** a conventional capsule containing the same control cell line being K562 human erythroleukemia cells expressing human GM-CSF; **B**: GM-CSF release from explanted capsules that were subcutaneously implanted in mice for one week (same groups as in Figure A); **C:** GM-CSF level in serum of mice implanted with capsules for one week (same groups as in Figure A); D: Quantity of GM-CSF detected in the subcutaneous tissue around the implanted capsule after one week (same groups as in Figure A).

### Detailed description

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it such as a preventive early asymptomatic intervention; (b) inhibiting the disease, i.e., arresting its development; or relieving the disease, i.e., causing regression of the disease and/or its symptoms or conditions such as improvement or remediation of damage. In particular, the methods, uses, formulations and compositions according to the invention are useful for encapsulated cell therapy.

The term "subject" as used herein refers to mammals. For examples, mammals contemplated by the present invention include human, primates, domesticated animals such as cattle, sheep, pigs, horses, laboratory rodents, other pets and the like.

The term "effective amount" as used herein refers to an amount of at least one compound of the invention or a pharmaceutical formulation thereof according to the invention that elicits the biological or medicinal response in a tissue, system, animal or human that is being sought. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. The term also includes herein the amount of compound of the invention sufficient to reduce the progression of the disease, notably to reduce or inhibit the progression of a xxx disorder and thereby elicit the response being sought (i.e. an "effective amount").

The term "efficacy" of a treatment according to the invention can be measured based on changes in the course of disease in response to a use or a method according to the invention. For example, the efficacy of a treatment of cancer can be measured by a reduction of the tumor size or by an increase of the overall survival (OS), or of the progression free survival (PFS), or by obtaining disease stabilization (SD), or a decrease in serum tumor markers such as Prostate specific antigen (PSA), Cancer antigen 125 (CA125), Carcinoembryogenic Antigen (CEA), or a reduced metabolic activity, reduced genetic abnormalities such as circulating tumor DesoxyriboNucleicAacid (ctDNA).

The term "myogenic potential" refers to the ability of the cells to form myotubes. This ability can be tested by standard techniques such as described herein or by flow cytometry, western blotting.

The term "human myoblast phenotypic marker" refers to a phenotypic feature for human myoblasts such as CD56+, CD146+, CD82+. Those can be assessed by Fluorescence-activated cell sorting (FACS) or by immunohistochemistry or western blot. According to a particular aspect, the said at least one myoblast phenotypic marker is selected from CD56+, CD146+ and CD82+.

The term "cell growth medium" refers to a medium suitable for growing myoblasts such as a culture medium supplemented with at least one growth factor such as epidermal growth factor, creatin, uridin, dexamethasone, fetal bovine serum, fetuin, bovine serum albumin, insulin or pyruvate. According to a particular embodiment, amplification medium may refer to a cell growth medium as defined above.

The term "pharmaceutical formulation" refers to preparations which are in such a form as to permit biological activity of the active ingredient(s) to be unequivocally effective and which contain no additional component which would be toxic to subjects to which the said formulation would be administered.

### Method of preparation of immortalized human myoblast cells according to the invention

According to one aspect, the invention provides a method of establishing an immortalized human myoblast cell line, said method comprising the steps of:
a) Transducing at least one primary human myoblast cell with a lentiviral vector forencoding the cyclin-dependent kinase 4 (CDK4) gene and a lentiviral vector encoding for the catalytic subunit of the human telomerase (hTERT) gene to obtain immortalization of the said primary human myoblast cell;
b) Growing cells from at least one immortalized primary human myoblast cell obtained under step a) in a myoblast cell growth medium and isolating from the growth medium each obtained cell presenting at least one myoblast phenotypic marker in a separate culture medium;
c) Growing separately in individual culture and amplification media each isolated cell obtained under step b);
d) Selecting among all the individual culture and amplification media of step c) at least one cell line with improved stability or expression properties from the parental cell by single cell cloning;
e) Controlling the myogenic potential of the selected at least one cell line.
f) Selecting individual clones based on their ability to survive in an encapsulation device;
g) Optionally repeating steps c) to f) sequentially to further improve the selected line.

According to a particular aspect, step a) of a method of the invention can be carried out using two separate lentiviral vectors or one single bicistronic vector such as described in Reiser et al, 2000, J Virol. 2000, 74(22): 10589-10599*;* Amendola et al, 2005, Nat Biotech, 23, 108-116*.*

According to a particular aspect, the lentiviral vectors used under step a) are vectors of pCLX type such as described in Salmon, 2013, Methods Mol Biol, 945, 417-448*.*

According to a particular aspect, the said at least one primary human myoblast cell provided for transduction under step a) can be isolated from human muscle tissue by standard methods such as enzyme digestion as described in Laumonier et al., 2017, J Vis Exp., 26 (125*).*

According to a further particular aspect, the growth of the cells under step b) until non-immortalized cells have died.

According to a further particular aspect, the growth of the cells under step b) is carried out for at least 1.5 month, i.e. the time necessary for non-immortalized cells to die.

According to a particular aspect, the myoblast selected under step b) of a method of the invention present myoblast phenotypic markers CD56+, CD146+ and CD82+.

According to a particular aspect, step c) is carried out until the cells show stable proliferation and viability when maintained in culture over 4 weeks.

According to another aspect of the invention, is provided a method for the preparation of genetically engineered immortalized human myoblast cells expressing a therapeutic protein under hypoxic conditions, wherein said method comprises the steps of:
i) Providing at least one immortalized primary human myoblast cell;
ii) Transducing said at least one immortalized primary human myoblast cell with a lentiviral vector for the expression of a target protein under the control of a phosphoglycerate kinase (PGK) promoter;
iii) Growing separately in individual culture and amplification media each isolated cell obtained under step ii);
iv) Selecting among all the individual culture and amplification media of step iii) at least one cell line with the highest secretion level for the target protein such as by ELISA or western blotting;
v) Controlling the myogenic potential of the selected at least one cell line.

According to a particular aspect, the at least one cell line selected under step iv) is a cell line presenting a secretion level of at least 1pg/cell/day of the target protein.

According to a particular aspect, the target protein is human GM-CSF.

According to another aspect, the invention provides a method for the treatment by immunotherapy of a subject, said method comprising administering at least one genetically engineered immortalized human myoblast cell according to the invention in said subject in need thereof.

According to another aspect, the invention provides a method for the treatment of a cancer in a subject, said method comprising administering at least one genetically engineered immortalized human myoblast cell according to the invention in said subject.

### Cells according to the invention

According to a particular aspect of the invention, is provided an immortalized human myoblast cell line or a composition comprising immortalized human myoblast cells or a progeny thereof derived from primary human myoblast cells, wherein said cells express CDK4 and hTERT and retain myoblast features such as ability of differentiation into myotube According to another aspect of the invention, the immortalized human myoblast cell line according to the invention present a proliferation rate corresponding to a doubling time of about 24 to about 72 hours for at least 6 months.

According to another aspect of the invention, the immortalized human myoblast cell line according to the invention present a survival term of at least about 48 hours under hypoxic conditions in standard myoblast culture medium.

According to a particular aspect of the invention, is provided immortalized human myoblast cells according to the invention are able to continue growing stably for up to at least six months in myoblast culture medium.

The immortalized human myoblast cells according to the invention have demonstrated a prolonged survival in an encapsulation device while maintaining a secretion of high levels of proteins interest and therefore offer a unique and very advantageous platform for further developing new cell lines genetically engineered to secrete proteins of interest in various domains of application.

For example, the genetically engineered immortalized human myoblast cells are typically able to secrete a protein at a rate of about 1 for about 15 pg/cell/day under standard myoblast culture conditions.

According to another aspect of the invention, is provided a genetically engineered immortalized human myoblast cell line or a composition comprising genetically engineered immortalized human myoblast cells or a progeny thereof, wherein said cells express CDK4, hTERT and a therapeutic protein, retain myoblast features and are able to secrete said therapeutic protein.

For example, genetically engineered immortalized human myoblast cells according to the invention express GM-CSF and are able to secrete a GM-CSF. Typically, according to a particular aspect, genetically engineered immortalized human myoblast cells according are able to secrete GM-CSF at a rate of about 1 to about 5 pg/cell/day for about 6 months under standard myoblast culture conditions.

According to another aspect of the invention, is provided a genetically engineered immortalized human myoblast cell line expressing human, humanized or chimeric monoclonal antibodies (e.g. the anti-CD20 monoclonal antibody rituximab) or recombinant proteins (e.g. mouse or human hormones or growth factors).

### Compositions according to the invention

The invention provides cells and pharmaceutical compositions thereof and methods for treating a subject, preferably a mammalian subject, and most preferably a human patient who is suffering from a medical disorder.

In another embodiment, the invention provides a pharmaceutical composition containing at least one cells of the invention and a pharmaceutically acceptable carrier, diluent or excipient thereof.

Immortalized cells of the invention or formulations thereof may be administered as a pharmaceutical formulation, which can contain one or more agents according to the invention in any form described herein. For use as encapsulated cells, the cells may be prepared as various types of suspension or other fluid formulation in a cell growth medium, preferably an animal-free medium.

In one embodiment, the cells may be suspended in a suitable fluid medium, such as a growth or differentiation medium. The fluid medium may comprise a physiologically acceptable aqueous solution for the growth or maintenance of the living cells. For example, the fluid medium may comprise glucose, salts, minerals, buffers, amino acids, hormones and growth factors that the cells need and use *in vitro* and/or *in vivo.* Suitable fluid media for encapsulated cells are described, for example, in PBS, HBSS, MyoCult or cell growth medium as described herein.

In one embodiment, the cells are encapsulated in hydrogels such as polyethylene glycol (PEG), alginate or chitosan hydrogels. PEG-based encapsulation is described in more detail in Lathuillère et al., 2014, Biomaterials 35 780-790. Cells can grow on supporting matrices such as polyvinyl alcohol (PVA), PEG, polyethylene, polyesther such as described in Li, 2000, Tissue Eng., 6(2):151-63*;* Li, 1999, Tissue Eng., 5(5):453-66*;* Uludag, 2000, Adv Drug Deliv Rev., 42(1-2):29-64*.*

In one embodiment, the cell encapsulated in a device that needs to be cryopreserved may be prepared in a formulation containing cryoprotectant such as described in Elliott et al., 2017, Cryobiology; 76:74-91*.*

### Implantable devices or kits comprising cells of the invention

Immortalized myoblast cells of the invention can be used for ECT and therefore can be encapsulated in cellular implants such as in flat sheets as described in *Lathuillère et al., 2014, supra* or WO 2014/173441 or in hollow fiber devices such as described in *Lathuillère et al., 2015, supra,* depending on the secreted protein and the site of implantation of the device. Alternative devices such as described in Orive et al., 2019, Prog Retin Eye Res, 68:67-82 may also be used.

According to a particular aspect, is provided an implantable device containing an effective amount of immortalized human myoblast cells of the invention.

According to another particular aspect, is provided a kit for cell based therapy in a mammal, comprising an effective amount of immortalized human myoblast cells of the invention and instructions for the use thereof to prepare a cell encapsulation implant.

According to another particular aspect, is provided a kit for cell based therapy in a mammal comprising an implantable device containing an effective amount of said immortalized human myoblast cells and instructions for the use thereof.

According to a further embodiment, a kit of the invention may further comprise antigenic material (e.g. one or more antigen(s)) useful for anti-cancer or anti-infection vaccination.

According to a particular embodiment, the cell chamber of an implantable device contains between 1.0 x 10⁴ cells and 8 x10⁵ immortalized human myoblast cells according to the invention (e.g., 1.0 x 10⁴, 5.0 x 10⁴, 1.0 x 10⁵, 3.0 x 10⁵, 5.0 x 10⁵ or 8 x 10⁵ or 10⁶ cells), depending on the application and the secreted protein. Those skilled in the art will recognize that the exact cell number in each chamber can depend both upon the growth rate of the cell/cell line encapsulated and/or the volume of the individual chambers used to construct the device

According to a particular embodiment, immortalized human myoblast cells secreting GM-CSF can be conditioned in an implantable capsule as described in WO 2017/064571 in view of a use in a personalized anti-tumor cell immunotherapy as described therein.

According to a particular embodiment, an implantable device containing at least one immortalized human myoblast cell according to the invention can be cryopreserved and cryopreserved devices can be transported under vapor phase liquid nitrogen (e.g., -190 ° C) conditions and/or under dry ice (e.g., -70° C) conditions using any method(s) known in the art.
Before implantation, cryopreserved devices can be thawed using any method(s) known in the art, typically in a 37°C water bath or dry bath. before implantation.

According to a particular aspect, the viability and/or functionality of the cells prior to encapsulation and/or prior implantation after thawing can be carried out to confirm their suitability for use, e.g., in transplantation. This can be accomplished using a variety of methods known in the art. For example, the cells can be stained using vital stains, such as, e.g., trypan blue or ethidium bromide or acridine orange or The Live/Dead assay kit (Molecular Probes; Thermo Fisher Scientific, Waltham, MA). In preferred embodiments, a population of cells suitable for transplantation is at least about 50%, at least about 75%, at least about 95%, or at least about 99%, viable. In other embodiments, the morphometric characteristics of the cells can be determined as a measure of the suitability of cells for use in implantation.

### Mode of administration

Cells and formulations thereof according to this invention may be administered by implantation of a biocompatible implantable device containing an effective amount of immortalized human myoblast cells of the invention which allows slow release of the protein secreted by the living cells inside the implant.
According to another aspect, cells and formulations thereof according to this invention may be administered in microcapsules or microspheres such as described in Acarregui et al., 2013 Biomacromolecules, 14(2), 322-330 or in hydrophobic matrices or hydrophilic matrices, biodegradable matrices or mineral matrices.
According to another aspect, cells and formulations thereof according to this invention can be directly injected as cell suspensions.

According to a particular aspect, an implantable device suitable for the cells of the invention can be as follows:
Disclosed herein is an implantable capsule comprising a cell receiving portion comprising a porous membrane surrounding a cell receiving chamber for receiving immortalized cells in a liquid media therein for the secretion of therapeutic agents, characterized in that the capsule further comprises a cell support matrix inserted within the cell receiving chamber configured for the arrangement of the immortalized cells within the cell receiving chamber.

In an embodiment, the cell support matrix comprises at least one yarn.

In an advantageous embodiment, said at least one yarn consists of or comprises a polyester material.

In an advantageous embodiment, the cell support matrix comprises a plurality of said yarns.

In an advantageous embodiment, the plurality of yarns is in a range of 5 to 20 yarns, preferably a range of 5 to 15 yarns, for instance around 10 yarns.

In an advantageous embodiment, the yarns extend within the cell receiving chamber over substantially the whole length of the chamber or at least 80 per cent of the length of the cell receiving chamber.

In an advantageous embodiment, the cell receiving chamber comprises polyester yarns.

In an advantageous embodiment, the cell receiving portion further comprises a membrane support mounted in the cell receiving chamber configured for providing structural support to the porous membrane, the membrane support consisting of or comprising a coil made of a biocompatible material, for instance of stainless steel coil.
In an advantageous embodiment, the capsule further comprises an extractor portion coupled to an extractor end of the cell receiving portion configured for allowing a surgical tool to pull the implantable capsule out of an implantation site, the extractor portion comprising a retrieval thread.
In an advantageous embodiment, the retrieval thread is made of a thread of polypropylene.
In an advantageous embodiment, the extractor portion comprises an anchor tube (8) having a cavity therein within which an anchor portion (15) of a retrieval thread (9) is inserted and bonded.
In an advantageous embodiment, the anchor tube consists of or comprises a polyurethane material.
In an advantageous embodiment, the extractor portion is coupled to the cell receiving portion via a coupling comprising a connector, a connector comprising a portion inserted into an extractor end of the porous membrane and second portion inserted in a coupling end of the anchor tube.
In an advantageous embodiment, the connector is bonded to the anchor tube and the cell receiving portion by an adhesive, in particular a light curable adhesive, for instance of the type photocurable urethane methacrylate.
In an advantageous embodiment, an outer diameter of the capsule is in a range of 0.5 to 3 millimeters preferably in a range of 0.8 to 1.5 millimeters and has a length in a range of 5 to 25 millimeters preferably in a range of 8 to 20 millimeters.
In an advantageous embodiment, a length to diameter ratio of the capsule is in a range of 5 to 20.

Referring to figure 9, an implantable capsule 1 according to an embodiment of invention comprises a cell receiving portion 2 and an extractor portion 3 connected together by a coupling 4. The cell receiving portion 2 comprises a substantially cylindrical outer shape with a diameter that may typically be in the range of 0.5 to 3 millimeters and a length that may be typically in the range of 5 to 20 millimeters for instance around 10 millimeters. The ratio *L*/*D* of the length *L* to the diameter *D* is preferably in a range from 5 to 20 preferably in a range from 5 to 15. The capsule may be implanted in a patient's tissue by means of implantation devices that are *per se* well-known in the field of implants and need not be further described herein.

The cell receiving portion 2 comprises a porous membrane 5 that is configured for allowing therapeutic agents produced by the cells 24 contained in the capsule through the membrane to the surrounding tissue and to allow fluids and electrolytes and nutrients for the cells 24 to pass into the capsule through the membrane from the surrounding tissue. The porosity and the type of membrane may thus depend on the specific application and type of cells that are contained within the capsule. In an example, the membrane is for instance in a form of a polyethersulfone (PES) membrane having a porosity around 0.65µm configured to allow the target molecules to pass though the membrane. An example of a membrane may be used in the present invention is detailed hereafter:
An exemplary embodiment of a membrane comprises polyethersulfone based on its biocompatible chemical composition, the structure properties as well as the inherent membrane performance, such as the superior flow rates, the downstream cleanliness and the low protein binding affinity. It can be extruded in different shapes and in small diameter tubing.
The cell receiving portion 2 can have the form of a flat sheet as described in Lathuillère et al., 2014, Biomaterials 35 780-790 or WO 2014/173441 or hollow fiber such as described in *Lathuillère et al., 2015,* supra, depending on the secreted protein and the site of implantation of the device.
The cell receiving portion 2 may contain an effective amount of cells such as for example between about 1.0 x 10⁴ cells and 8 x10⁵ cells according to interest (e.g., 1.0 x 10⁴, 5.0 x 10⁴, 1.0 x 10⁵, 3.0 x 10⁵, 5.0 x 10⁵ or 8 x 10⁵ or 10⁶ cells), depending on the application and the secreted protein. Those skilled in the art will recognize that the exact cell number in the cell receiving portion can depend both upon the growth rate of the cell/cell line encapsulated and/or the volume of the cell receiving portion.
The porous membrane 5 surrounds a cell receiving chamber 13 and a membrane support 6 within the cell receiving chamber 13. The membrane support serves to mechanically support the porous membrane to maintain the stability of the volume in the cell receiving chamber 13 and to prevent rupture of the membrane. In the illustrated embodiment, the membrane support is in a form of a coil, in particular a stainless steel coil that is *per se* known for instance as described in WO 2017/0645701. The membrane support 6 also serves to anchor the extractor portion 3 via the coupling 4.

In the illustrated embodiment, the coupling 4 comprises a connector 10 having a portion 10a that is inserted into the membrane support 6, in particular within the cylinder surrounded by the stainless steel coil in the present example. The diameter of the connector insert portion 10a may be configured to engage in a tight fit in an extractor end of the coil for a stable connection therebetween. The coupling 4 further comprises a fixing portion 10b engaging an anchor 8 of the extractor portion 3, whereby in the presently illustrated embodiment the anchor 8 is in the form of a tube, preferably a polyurethane (PU) tube fitted over the second end 10b of the connector 10. An adhesive 18a may be deposited on the connector prior to insertion of the exactor end 12b of the cell receiving portion, respectively coupling end 8a of the anchor to 8 on the connector 10. The adhesive may advantageously be in the form of a light curable adhesive for instance of the type photocurable urethane methacrylate (such as dymax 1187 M SV).
The extractor portion 3 serves to provide a means to pull out the implant from the patient's tissue at the end of its use. In the illustrated embodiment, the extractor portion further comprises a retrieval thread 9 comprising an anchor portion 15 fixed to the anchor tube 8 and a thread portion 16 extending beyond the anchor tube configured for allowing a surgical device to catch the thread to pull out the implantable capsule. In the illustrated embodiment, the retrieval thread 9 is made of a biocompatible yarn or thread, for instance of the type Polypropylene (such as the Prolene ™ suture).In an embodiment, a length of the thread extends within the hollow anchor tube 8 and comprises knots 15a, the anchor portion 15 being held within the tube by an adhesive, for instance a light curable adhesive as described above, the knots increasing the strength of the attachment of the retrieval thread to the anchor tube. The retrieval thread is thus supple and very fine to reduce discomfort of the patient and allow easy removal of the implant.
It may be noted that the extractor portion 3 may have different shapes and configurations, the purpose being to allow a surgical device to grip on to the implant and to pull it out of the patient's tissue.
In a variant, the retrieval thread may be directly fixed to the connector 10, or be integrally formed with the connector 10, without the presence of the anchor tube. In such a variant the connector may for instance comprise an orifice allowing a thread to feed through the orifice and allow for pulling the implanted capsule out of the patient's tissue. The polyurethane tube, or any other material with the adequate mechanical and biological properties, advantageously provides a structure which supports the coupling of the retrieval thread. It may also be used as the support for the tweezers during manipulations, whether it is during the assembly or the implantation.
The cell receiving portion 2 further comprises a cell support matrix 7 inserted within the cell receiving chamber. In a preferred embodiment, the cell support matrix 7 comprises one or more yarns 14 of a biocompatible material, preferably a plurality of yarns extending longitudinally within the cell receiving chamber 13. The yarns extend, in a preferred embodiment, from a position at or proximate the extractor end 12b to a position at or proximate a cell loading end 12a of the membrane 5. The yarns preferably extend over the entire length or a large portion of the length of the cell receiving chamber 13. In a preferred embodiment, the yarns may advantageously be made of polyester (PE) of clinical grade that is *per se* well-known and already approved for surgical implantation uses. Such polyester yarns are typically used for sutures of tissue within a patient's body. An example of polyester yarns that may be used in advantageous embodiments of the invention is for instance 44/27-PET-5540-FTT-SS (Textile Development Associates, Inc). The material is 40 denier 27 filaments yarn made of textured polyester.
It has been found that the cell support matrix 7 significantly improves the performance of the immortalized cells contained in the cell receiving chamber increasing both activity in a release of therapeutic agents and durability over time, in particular for adherent cells. Such adherent cells are for instance genetically engineered cells useful in cell therapy such as for example genetically engineered immortalized human myoblasts, mouse myoblasts, human retinal pigment epithelial cells, stem cells, stem cell derived cell lines. whereby it is being found that these cells 24 tend to align with the fibers of the yarn 14 thus improving the density and spacing of the cells optimized for release of therapeutic agents and ingestion of nutriments. The yarns also advantageously provide a large overall surface area for adherents of cells thereto.
In an exemplary embodiment, immortalized human myoblast cells secreting GM-CSF are loaded in the cell receiving portion 2 of a capsule of the invention. Those capsules are useful in a personalized anti-tumor cell immunotherapy.
The cells are loaded in the cell receiving portion in a cell growth medium suitable for the type of cells such as Ham's F12 or DMEM supplemented with growth factors or fetal bovine serum. Further, for cells that will be frozen, a freezing medium/cryopreservant is also added to the cell growth medium, such as for glycerol.
In an exemplary embodiment, a cell receiving cavity 13 may receive therein for instance five to twenty yarns 14 arranged in parallel within the cavity extending substantially the whole length of the cavity. The yarns may be inserted within the cell receiving cavity 13 by pulling one end thereof through the cavity, the coupling 4 being mounted on the extractor end 12b of the cell receiving portion 2 once the membrane support 6 and cell support matrix 14 have been mounted in the porous membrane 5.
It may be noted that the cell support matrix 7 may be preassembled to the membrane support 6, for instance by inserting them through the inside of the coil prior to the insertion of the preassembled coil and cell support matrix into the tubular porous membrane 5.
The cell support matrix 7, in particular in the form of yarns 14, thus has a very beneficial effect of optimizing the distribution of cells in an orderly manner within the cell receiving chamber, improving the secretion yield and rate for a given volume. Moreover, this configuration allows the length of the cell receiving portion to be easily modified by simply changing the cut length of the yarns to the corresponding length of the porous membrane tube and coil of the membrane support 6. Moreover, the use of well characterized biocompatible implantable polyester does not adversely impact the safety of the device.
It has also been observed that the presence of the cell support matrix 7 allows the freezing and defreezing of cells contained without affecting the viability of the cells. The presence of the matrix 7 improves the freezing and defreezing properties of the capsule which is particularly advantageous since it allows the capsules to be stored for extended periods of time in a frozen state, ready for use in the treatment of a patient when needed. In particular, it would appear that the improved distribution of cells, in particular adherent cells, along the yarns contribute to maintain a high rate of viability during the freezing and thawing process.
The cells in a liquid media may be inserted into the cell receiving chamber 13 by means of a cell loading device 20 (only partially and schematically represented in the illustrations) comprising an outlet nozzle 22 that is inserted in a cell loading end 12a of the porous membrane 5.
The implantable capsule 1 may be supplied in a preassembled arrangement with the cell loading device. In this embodiment, the nozzle 22 of the cell loading device may be attached to the cell loading end 12a of the membrane, for instance by means of an adhesive 18c, for instance a light curable adhesive as already described hereinabove. The cell loading device may comprise a catheter tube to allow the cells in a liquid media to be injected through the catheter tube into the cell receiving chamber of the capsule, air contained within the cell receiving capsule being pushed out through the porous membrane 5.

As noted above however, the capsule may be filled with the immortalized cells and the media in a ready to use state the cell receiving end 12 being hermetically sealed by a plug (not shown) and then frozen until needed for treatment of a patient.
An implantable device of the invention can be implanted into a living subject in various sites, including under the skin (e.g. subcutaneously). Alternatively, the device may be implanted at an intrathecal, intracerebral, intraosseous, intratumoral, intrapleural, intraocular or intraperitoneal site.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, cells and pharmaceutical formulations thereof can be administered alone or in combination with a co-agent useful for prevention and/or treating a cancer, in particular cancer cell antigens and/or immune check-point inhibitors such as PD-1, PD-L1 or CTLA4 inhibitors or a co-agent useful for prevention and/or treating autoimmune disorders.

The invention encompasses the administration of cells of the invention or a formulation thereof wherein it is administered to a subject prior to, simultaneously or sequentially with other therapeutic regimens or co-agents.

Cells of the invention or a formulation thereof according to the invention that is administered simultaneously with said co-agents can be administered in the same or different composition(s) and by the same or different route(s) of administration.

According to one embodiment, is provided a pharmaceutical formulation comprising at least one cell of the invention combined with at least one co-agent useful for prevention and/or treating a cancer and at least one pharmaceutically acceptable carrier.

### Subjects

According to another aspect, the cells, devices, kits and methods of the invention are useful for the treatment of a subject in need of a treatment by a therapeutic protein.

According to a particular aspect, provides cells, devices, kits and methods of the invention are useful for the treatment of a cancer.

According to another particular aspect, provides cells, devices, kits and methods of the invention are useful for a treatment by immunotherapy.
According to another particular aspect, provides cells, devices, kits and methods of the invention are useful for a treatment of an inflammatory disorder.
According to another particular aspect, provides cells, devices, kits and methods of the invention are useful for a treatment of neurodegenerative disorder.

For example, cells secreting the GM-CSF immunostimulatory cytokine can be usefully used as part of personalized anti-tumor cell immunotherapy as described in Gupta & al., 2010, Moving Forward. Discov Med., 50:52-60*.*

According to a particular aspect, immortalized cells and methods according to the invention may be used for the production of other proteins (e.g. antibody or antibody fragment) modulating the intensity of the immune response and in particular the blocking of CTLA-4 protein (immune check point inhibitor) for the treatment of a cancer, in particular melanoma.
For applications in the field of oncology, the following proteins may be useful to be secreted by encapsulated cells in the context of the invention: Trastuzumab (Herceptin®), Pertuzumab (Perjeta®) for breast cancers (HER2+), Rituximab (Rituxan®) for lymphomas and CML (chronic myelogenous leukemia), Blinatumomab (Biincyto®) for Acute lymphocytic leukemia (ALL), Obinutuzumab (Gazyva®), Ofatumumab (Arzerra®) for chronic lymphocytic leukemia (CLL), Cetuximab, (Erbitux®) for colon cancer and head and neck cancers, Panitumumab (Vectibix®) for colon cancer, Necitumumab (Portrazza®) for lung cancer, Bevacizumab (Avastin®) for colon cancer or relapsing brain tumours, Ramucirumab (Cyramza®) for stomach cancer, Nivolumab (Opdivo®), Pembrolizumab (Keytruda®), Atezolizumab (Tecentriqu®), Durvalumab (lmfizi®), Avelumab (Bavencio®) for advanced cancers such as melanoma, lung, head and neck, bladder and kidney cancers, lpilimumab (Yervoy®) for melanoma and kidney cancer, Daratumumab (Darzalex®) or Elotuzumab (empliciti®) for multiple myeloma, Dinutuximab (Unitixin®) for neuroblastoma, Olaratumab (Lartuvo®) for soft-tissue sarcomas and Catumaxomab (Removab®) for refractory ovarian cancer.
For applications in the field of inflammatory disorders, the following proteins may be useful to be secreted by encapsulated cells in the context of the invention:
Adalimumab (Humira®), Infliximab (Remicade®), Golimumab (Simponi®) for colon inflammatory disorders, rheumatoid polyarthritis, ankylosing spondylarthritis polyarthrite rhumatoide, and psoriasis, Belimumab (Benlysta®) for systemic lupus erythematosus, Tocilizumab (Actemra®), Sarilumab (Kevzara®) for rheumatoid polyarthritis or juvenile polyarthritis, Brodalumab (Siliq®), lxekizumab (Taltz®), Secukinumab (Cosentyx®) for psoriasis, Guselkumab (Tremfya®) for psoriasis, Ustekinumab (Stelara®) for psoriasis and Crohn's disease, Vedolizumab (Entyvio®) for ulcerative colitis and Crohn's disease, Canakinumab (llaris®) for cryopyrinassociated periodic syndrome, Daclizumab (Zinbryta®) or Natalizumab (Tysabri®), Ocrelizumab (Ocrevus®) for multiple sclerosis,, Dupilumab (Dupixent®) atopic dermatitis, Mepolizumab (Nucala®), Reslizumab (Cinqai®) and Benralizumab (Fasenra®) for asthma, Ranibizumab (Lucentis®) for macular degeneration. For applications in other therapeutic fields, therapeutic proteins, in particular insulin or antibodies such as described in Kaplon et al, 2019, Mabs, 11(2):219-238*.*

### Methods and uses according to the invention

The invention provides genetically engineered immortalized human myoblast cells according to the invention for use in the prevention and/or treatment of a disorder or a disease, in particular a cancer, an inflammatory disorder or a neurodegenerative disorder.
The invention further provides the use of genetically engineered immortalized human myoblast cells according to the invention for the preparation of a pharmaceutical preparation (e.g. encapsulated cell preparation) useful the prevention and/or treatment of a disorder or a disease, in particular a cancer, an inflammatory disorder or a neurodegenerative disorder.
The invention further provides a method of preventing or treating a disorder or a disease associated in a subject, in particular a cancer, an inflammatory disorder or a neurodegenerative disorder, said method comprising administering in a subject in need thereof a therapeutically effective amount of genetically engineered immortalized human myoblast cells of the invention.
According to one aspect, the genetically engineered immortalized human myoblast cells according to the invention are useful in the treatment of a cancer (for example). For example, the genetically engineered immortalized human myoblast cells according to the invention are secreting GM-CSF.
In particular, the genetically engineered immortalized human myoblast cells according to the invention are useful in the treatment of a cancer.
According to another aspect, the genetically engineered immortalized human myoblast cells according to the invention are useful in the treatment of an inflammatory disorder (e.g. rheumatoid arthritis). For example, the genetically engineered immortalized human myoblast cells according to the invention are secreting an anti-TNF alpha antibody.

References cited herein are hereby incorporated by reference in their entirety. The invention having been described, the following examples are presented by way of illustration, and not limitation.

### EXAMPLES

The following studies are conducted to support the effectiveness of cells and methods of the invention according to the invention.

### The following abbreviations refer respectively to the definitions below:

**DAPI** (4,6 Diamidino-2-phenylindole); **DMEM** (Dulbecco's Modified Eagle Medium); **DPBS** (Dulbecco's phosphate-buffered saline); **EDTA** (ethylenediamine tetraacetic acid); **FBS** (Fetal Bovine serum); **HBSS** (Hank's Buffered Salt Solution), **MEF** (myocyte enhancer factor); **PFA** (paraformaldehyde).

### Example 1: Preparation of an immortalized human myoblast cell line

An immortalized human myoblast cell line has been prepared according to a method according to the invention as detailed below:
The following reagents were used:

### Myoblast Growth Medium (GM):

Ham's F10 (GIBCO 41550021) supplemented with
- 15% FBS (GIBCO 10101145)
- Bovine serum albumin (Sigma-Aldrich A4503; 0.5 mg/ml)
- Fetuin (Desert Biological 302070; 0.5 mg/ml)
- Epidermal growth factor (R&D Systems 236-GMP-200; 10 ng/ml)
- Dexamethasone (PharmaServ 8016; 0.39 µg/ml)
- Insulin (Sigma-Aldrich 19278; 0.04 mg/ml)
- Creatine monohydrate (ParmaServ 8114; 149 µg/ml)
- Pyruvate (Gibco 11360039; 100 µg/ml)
- Uridine (U3003; 50 µg/ml)
- Gentamycin (Gibco 15710049; 5 µg/ml)

### Myoblast Differentiation Medium (DM):

DMEM (GIBCO 61965026) supplemented with
- Bovine serum albumin (Sigma-Aldrich A4503; 0.5 mg/ml)
- Epidermal growth factor (R&D Systems 236-GMP-200; 10 ng/ml)
- Insulin (Sigma-Aldrich 19278; 10 µg/ml)
- Creatine monohydrate (ParmaServ 8114; 149 µg/ml)
- Pyruvate (Gibco 11360039; 100 µg/ml)
- Uridine (U3003; 50 µg/ml)
- Gentamycin (Gibco 15710049; 10 µg/ml)

### Blocking Solution:

DPBS (Sigma D8537) with
   - Goat Serum (Sigma G9023, 2%)
   - Tween 20 (AppliChem A1389, 0.2%)
HBSS (GIBCO 14175053)
PFA 4% (Santa Cruz sc-281692)
Triton X-100 (AppliChem A1388)
Liquid blocker (Arcus Biologicals NAN-012)
Ab Mouse anti MF20 (Hybridoma Bank)
Ab Rabbit anti MEF2 (Santa Cruz sc-313)
Ab Goat anti-Mouse Alexa 488 (Life A11029)
Ab Goat anti-Rabbit Alexa 546 (Life A11035)
DAPI Fluoromount-G (SouthernBiotech 0100-20).

### 1.1 Human myoblast cell source

Human myoblast cells were obtained from human muscle tissue obtained under informed consent as small muscle debris dissected during a reconstruction surgery from a non-smoking 32-year-old female donor with no significant medical history or chronic disease suffering from a left knee traumatism with anterior cruciate ligament injury. The eligibility criteria for tissue donation were defined according to the FDA guidelines "Eligibility Determination for Donors of Human Cells, Tissues, and Cellular and Tissue-Based Products (HCT/Ps)". Criteria were the following:
- Age > 18 years
- Healthy subject: no identified chronic condition, including muscle disease
- Planned orthopaedic surgery
- Negative screening tests for the following infectious disease: HIV type 1 & 2, HBV, HCV, Treponema pallidum, HTLV 1 & 2, West Nile virus.
and the study protocol was approved by the Ethics Committee in Geneva.

### 1.2 Isolation of primary myoblast cells and growing step

Under sterile conditions in cell culture hood, the human muscle tissue was rinsed with DMEM. Fat and fibrotic tissue was removed with a pincet and scissors. The tissue was placed in a petri dish with 5 ml of Trypsin EDTA 0.05% and cut into millimeter pieces with scissors.

Pieces of muscle with trypsin were transferred in a sterile dissociation bottle. Volume of Trypsin EDTA 0.05% was completed to 90 ml for a maximum of 3g of tissue. Tissue was incubated at 37°C for 60 min under agitation. Digestion was stopped with 10% FBS. Muscle solution was then filtered through a 70 µm cell strainer and centrifuged at 1000 rpm for 5 min at room temperature (RT) to pellet the cells. After the supernantant was discarded, the pellet was dissolved into DMEM and filtered through a 40 µm cell strainer. 200'000 cells were plated per 60 mm Petri dish in 3.5 ml of growth medium (GM). Cells were incubated at 37°C and 5% CO₂. Once cells reached 75% confluency, GM was removed and cells were washed with HBSS. A minimum volume of Trypsin EDTA 0.05% was applied to cover the surface of the cells and incubated for 3 min at 37°C. Reaction was stopped with the same volume of GM. Cells were collected and centrifuged at 1000 rpm for 5 min at RT. Supernatant was discarded, cells were washed with GM and centrifuged again. For cell staining, cells were resuspended in 200 µl of GM and following antibodies were added: 3 µl of anti-CD56-AlexaFluor488, 0.5 µl of anti- CD82-PE, 3 µl of anti- CD146-PECy7. After a 30-minute-incubation at 4°C, cells were washed and sorted by flow cytometry using a MoFlo Astrios EQ (Beckman Coulter). Myoblasts were defined as CD56+ CD146+ CD82+.

### 1.3 Myoblast immortalization using lentiviral vectors encoding for CDK4 and hTERT (steps a) and b))

The immortalization of the myoblast cells obtained above was achieved by the transduction of 2 genes, the cyclin-dependent kinase 4 (CDK4) and the catalytic subunit of the human telomerase (hTERT) as earlier proposed (Zhu et al., 2007, Aging Cell, 6, 515-523). Transgenes were cloned into a third generation pCLX lentiviral vector backbone under the expression of the human phosphoglycerate kinase (hPGK) promoter as described in Salmon, 2013, Methods Mol Biol., ;945:417-48*.* Specific viral infectivity was titrated on HeLa target cells and expressed in transducing unit per ml (TU/ml) *(*Barde et al., 2010, Curr Protoc Neurosci, Chapter 4, Unit 4 21, 10.1002/0471142301.ns0421s53*).*

On day 0, primary human myoblasts were seeded at 20'000 to 30'000 cells per well in a 24 wells plate in 500 µl of GM. On day 1, cells were infected with lentivirus. To achieve immortalization, a multiplicity of infection (MOI) of 3 (number of viral copies/cell) for each vector (pCLX-PGK-hTERT & pCLX-PGK-CDK4 as described herein) was applied to the cells. On day 2, 500 µl of fresh GM was added in each well. On day 3, the cells were passaged in a new culture dish.

Cells were maintained in culture under 5% CO₂ at 37°C and proliferation was quantified using an automated cell counter (Countess, ThermoFischer). The number of population doublings at each passage was defined as log N/log 2, where N is the number of cells harvested at confluence divided by the number of cells initially seeded. While primary myoblast cells stopped proliferating after 45 days, cells that were transduced with lentiviruses continue growing stably for up to six months as can be seen under **Figure 1**.

This confirms that transduction of primary myoblast cells with lentiviral vectors encoding CDK4 and hTERT can efficiently immortalize cells.

### 1.4 Myoblast growing and cloning (steps c) and d))

Once cells reached 75% confluency, cells were detached, washed and stained with anti-CD56-AlexaFluor488, anti-CD82-PE and anti-CD146-PECy7 as previously described in *Laumonier et al., 2017, supra.* After a 30 minutes incubation at 4°C, cells were washed and sorted by flow cytometry using a MoFlo Astrios EQ (Beckman Coulter) in a 96-well plate with one cell per well. Myoblasts were defined as CD56+ CD146+ CD82+. Individual clones were then cultured in growth medium and amplified and sorted by sorting 1 cell/well in a 96 well plate from the immortalized myoblast cell population (I) using single cell cloning. A total of 33 clones were isolated, the proliferation rate of 9 selected clones that kept qualitative morphologic features (1-3, 5-6, 13, 17 and 20-21) was quantified using automated cell counting at each passage and proved to remain stable over time as can be seen on **Figure 2**.

### 1.5 Control of differentiation potential of myoblasts into myotubes (step e))

To evaluate the myogenic potential of myoblast clones obtained by a method of the invention after immortalization and sorting (after step d)), the maintenance of their ability to express myogenic markers over time (triple positivity for CD56, CD82 and CD146) was quantified by flow cytometry and their preserved capacity to fuse and differentiate into myotubes was quantified as follow. Cells were plated into 35 mm culture dish in GM. When cells reached 100% confluency, GM was removed, cells were washed with HBSS, and differentiation medium was added. After 72 hours, cells were washed with DPBS twice and fixed with PFA 4% for 15 min at 4°C. After 3 washes with DPBS a circle was drawn around cells with a water repellent pen. blocking solution was applied for 30 min at RT. Primary antibodies were incubated at 4°C overnight (mouse anti-MF20 diluted 1/1000 in blocking solution and rabbit anti-MEF diluted 1/300 in blocking solution). After 3 washes with DPBS and a 5 minutes incubation with blocking solution, secondary antibodies were incubated for 1 hour at RT (goat anti-mouse Alexa 488 diluted 1/1000 in blocking solution, goat anti-rabbit Alexa 546 diluted 1/1000 in blocking solution). After 3 washes with DPBS, coverslips were mounted with DAPI Fluoromount-G. For the quantification of differentiation percentage and fusion index, seven pictures per condition were randomly acquired at 20X on a fluorescence microscope. The number of MEF positive nuclei and DAPI nuclei was quantified.

As can be seen on **Figure 3**, the immortalized myoblast cells obtained by a method of the invention kept the myoblast phenotypic markers such as expression of myogenic markers (CD56, CD82 and CD146) and capacity to fuse and differentiate into myotubes. As can be seen, some clones demonstrated stable myogenic markers, other lost the expression suggesting lower stability and therefore, the clones presenting the best stabilities were used for further experiments. In particular, clone 61.27 deposited under number CCOS 1901 (derived from clone 2 above deposited under number CCOS 1902) was used for the encapsulation studies.

The obtained immortalized myoblast cell lines were characterized as follows in order to check their suitability for encapsulation technology:

### Myoblast encapsulation ability

The ability of the obtained immortalized myoblast cells to be encapsulated was assayed as follows: GM was removed and 75% confluent cells were washed with HBSS. A minimum volume of TrypLE (Thermo Fisher) was added to cover the surface of the cells and incubated for 3 min at 37°C. Cells were then gently detached from the dish surface. Reaction was stopped with the same volume of HBSS. Cells were collected and counted using an automated cell counter in duplicate (Countess II device, Thermo Fisher). Cells were centrifuged at 1'000 rpm for 5 min at RT and resuspended in 25 µl GM per 800'000-1'000'000 cells. 25 µl of cell suspension was loaded in a capsule containing a hollow fiber with an inner matrix as described in Figure 9 and Example 5 for genetically engineered immortalized myoblast cells for GM-CSF secretion. Immortalized human cells of hematopoietic origin, genetically modified to produce GM-CSF encapsulated in device as described in WO 2017/064571 (MVX-1) loaded in a capsule containing a hollow fiber as described in *Lathuiliere et al., 2015, supra* are used as control. The vasculon (loading tube of the capsule) was cut and the capsule was sealed using UV-sensitive adhesive (Dymax). The capsules were then placed in a 12-well plate in 2 ml of GM for 24h before implantation.

Based on their proliferation rate and differentiation capacity, 6 clones (#1, #2, #6, #13, #17 and #21) were selected for evaluation. These clones were encapsulated and kept in culture for up to 1 month. Capsules were then fixed and processed for histological analysis. Qualitative assessment of survival was then performed and this analysis concluded to good survival properties of clones #2 and #13 based on the living cell density inside the device (presence of living cells in the middle of the device and how the cells spread inside the device) which were then selected for genetic engineering for expression of GM-CSF as detailed below.

### Myoblast cell culture in hypoxic conditions

The behaviour of the obtained immortalized myoblast cells in hypoxic conditions was tested as follows: Cells were plated and cultured in standard conditions for 24 hours to allow for cell adhesion. Culture dishes were then placed in a hypoxic incubator chamber at 37°C with 1% oxygen for 24 hours. When recombinant protein needed to be quantified in culture medium, fresh medium that was pre-incubated for 24 hours in hypoxic chamber was added to the cells for two hours before quantification.

### Example 2: Alternative method of sorting the immortalized human myoblast cells under step c)

Alternatively, in a method of establishing an immortalized human myoblast cell line according to the invention, the isolation of immortalized human myoblast cell clones under steps b) and c) can be performed on encapsulated myoblast cell population according to steps b1) to b4).

Immortalized myoblast cell population obtained under 1.3 were encapsulated into an implantable device with an inner matrix as described on Figure 9.

The device were implanted in mouse subcutaneous tissue for 3 weeks. After 3 weeks, devices were retrieved and placed in culture medium. Capsules were sectioned and their content (cells and matrix) was disseminated in culture medium in a dish. Cells were kept in culture for several weeks for expansion. Cells were then detached and stained for the FACS sorting of human myoblast (defined as CD56+ CD146+ CD82+). Individual human myoblast clones were isolated from this myoblast population.

The myogenic potential (differentiation into myotubes) of 3 of these clones appeared unaffected. The proliferation rate of these clones was also monitored for several weeks and was a doubling time of about 24 to about 72 hours for at least 6 months.

### Example 3: Preparation of genetically modified immortalized human myoblast cell line

Immortalized human myoblast cell lines obtained by a method of the invention are useful for the preparation of genetically modified cells for the production of proteins of interest, in particular for their protein secretion within a capsule.

### 3.1 Myoblast transduction for the secretion of recombinant GM-CSF (step ii))

For the expression of human or murine GM-CSF, human or murine GM-CSF cDNA was cloned into the pCLX lentiviral vector under the expression of the human PGK promoter as described in *Salmon, 2013, supra.*
The human PGK promoter contains hypoxia response element which are enhancers that upregulate the gene expression in hypoxic conditions (Firth et al., 1994, Proc. Natl. Acad. Sci. U. S. A., 91, 6496-6500*).* To confirm this, primary myoblast cells with lentiviral vectors encoding for the GFP (MOI=5) under the expression of phosphoglycerate kinase (PGK) or ubiquitin (UBI) promoters and after 36h in 1% oxygen, cells were fixed and fluorescence was quantified by flow cytometry and it was observed that in myoblast cells, PGK promoter drives a stronger expression as compared to UBI and that myoblast cells upregulate the expression of GFP in hypoxic condition when driven by the PGK promoter as opposed to UBI promoter.
For this reason, the PGK promoter was used in the genetic engineering of immortalized human myoblasts of the invention since the secretion of target proteins might be enhanced when myoblast cells are encapsulated. Therefore, Clones #2 and #13 obtained in Example 1 were transduced with different concentration of the above lentivirus and different MOI (3-100) were applied to the cells.
The recombinant protein secretion ability by genetically modified immortalized human myoblasts was quantified as follows:
Cells were seeded in a T75 cell culture flask. Once 75% confluency was reached, cells were washed with HBSS and 5ml fresh growth medium was added for 2 hours at 37°C. Medium was then collected for GM-CSF quantification and cells were counted using an automated cell counter (Countess II device, Thermo Fisher). Different ELISA kits were used depending on the protein to be quantified (GM-CSF Human ELISA Kit #KHC2011, GM-CSF Mouse ELISA Kit #BMS612, IgG (Total) Human ELISA Kit #BMS2091, Thermo Fisher). Protocols were applied according to manufacturer instructions. Results were reported in pg/cell/day. As shown under **Figure 4A**, expression of GM-CSF was obtained from the genetically immortalized human myoblasts of the invention.

The secretion of GM-CSF from cell populations generated from transduced immortalized clone #2 was monitored weekly for nine weeks and it was confirmed that transduction with lentiviral vector is efficient to produce highly stable GM-CSF-secreting cell lines as shown under **Figure 4B**.

### 3.2 Isolation of human GM-CSF-secreting clones (steps iii) and iv))

From two GM-CSF expressing populations (Clones #2.100 & #13.10, 125 individual clones were sorted and expression of GM-CSF was measured for every clone. The 10 clones that secreted the highest levels of GM-CSF in a range of about 1-4 pg/cell/day for each population were monitored for their secretion of GM-CSF for several weeks (**Figure 4C**) and their proliferation (**Figure 5A**). Those data support that the secretion and proliferation rates of GM-CSF secreting clones remained stable over time.

The stability of the expression of myogenic markers over time (triple positivity for CD56, CD82 and CD146) for the isolated GM-CSF-secreting myoblast cell clones and their capacity to fuse and differentiate into myotubes was assessed as detailed above and confirmed as shown under **Figure 6**.

The obtained genetically modified immortalized myoblasts were characterized as follows in order to check their suitability for encapsulation technology:

### Myoblast encapsulation ability

The ability of the obtained immortalized myoblast cells to be encapsulated was assayed as described above for six clones selected based on their GM-CSF secretion level, growth rate and myogenic characteristics, i.e. the myogenic marker expression and the ability to differentiate into myotubes were encapsulated and then tested *in vivo* (mouse subcutaneous tissue).

### Implantation of hollow fiber encapsulation device in mice

All experiments on mice were done according to the Swiss regulation for the care and use of laboratory animals. Animals were bred and maintained in a specific pathogen free environment and had access to water and food ad libitum. Adult Rag2/Il2rg double knockout mice (Shinkai, 1992, Cell, 6;68(5):855-67) were anesthetized with isoflurane and capsules were implanted in the subcutaneous tissue using a trocar. Wound was closed with surgical staples and animals recovered in their home cage. Analgesia was provided by the subcutaneous injection of buprenorphine 0.1 mg/kg, 20 minutes before anaesthesia and acetaminophen 2 mg/ml in drinking water for three days. At the end of experiment, mice were sacrificed with a lethal injection of sodium pentobarbital. Devices were dissected and either fixed directly to perform histological analysis or placed back in culture medium for further quantification of recombinant protein expression. Histological processing was performed as previously described (Schwenter et al., 2011, Cancer Gene Ther., 18, 553-562).

The GM-CSF secretion level was measured from capsules before implantation and after explantation (at one and 3 weeks). GM-CSF was also measured in the mouse serum. Histological analysis was also performed on capsules after explantation to qualitatively measure cell survival. This experiment was performed using an encapsulation device with an inner matrix as described in Example 5 and **Figure 9**. The secretion levels were compared with those obtained with immortalized human cells of hematopoietic origin, genetically modified to produce GM-CSF encapsulated in device as described in WO 2017/064571 (MVX-1) and currently used in the current clinical trial n° NCT02193503 (phase I) NCT02999646 (phase II in HNSCC).
As it can be seen on **Figure 7**, all encapsulated clones presented much higher secretion levels than the MVX-1 cells, therefore confirming the advantageous effects of the immortalized cells of the invention, in particular for use in encapsulation therapy.

Then, Clone #61.27 deposited under number CCOS1901 (derived from clone 2 above deposited under number 1902) was selected based on his high secretion rate (about 3 pg/cell/day) and highest serum level of GM-CSF (suggesting high delivery of GMCSF by 61.27 clone) and clone #62.14 was selected because GM-CSF delivery remained stable over time, suggesting high cell survival rate. The proliferation rate of these two clones was monitored *in vitro* for 6 months and the GM-CSF secretion rate was also monitored *in vitro* for several months as non-encapsulated and encapsulated cells as described in Figure 8. **Figure 8** shows the stability over time of clones 61.27 and 62.14 in terms of *in vitro* proliferation rate as non-encapsulated cells **(A)** expressed as the number of cell population double (N) versus the number of weeks (W) after sorting of the clones from the genetically modified immortalized human myoblast population, *in vitro* secretion rate as non-encapsulated cells versus weeks (W) after sorting **(B)** and *in vitro* secretion rate as encapsulated cells versus weeks (W) after encapsulation **(C)** and *in vivo* secretion rate in mice as encapsulated cells measured by quantification of GM-CSF in serum compared before implantation **(D)** and in tissue surrounding the capsule **(E)**, compared to control encapsulated cells (MVX-1), as described in Example 3.
Encapsulation device as described in Example 5 and shown in **Figure 9** were loaded with those clones and secretion of GM-CSF was monitored for several months *in vitro* as described above compared to encapsulated cells (MVX-1) as defined above.

### Freezing and thawing of encapsulation devices pre-loaded with human myoblasts

The impact of different freezing conditions on the GM-CSF expression level after thawing was tested. Capsules were loaded with genetically modified GM-CSF secretion immortalized myoblast cells described (clones 61.27 and 62.14 in freezing medium that contained different concentration of glycerol (used as a cryopreservant). Before freezing, loaded capsules were suspended and incubated for variable periods of time in freezing medium. For freezing, capsules were placed in silicone tubes that were prefilled with freezing medium and transferred into cryotubes. Cryotubes were quickly transferred into CoolCell freezing container (Biocision) and placed at -80°C overnight before being transferred into liquid nitrogen for long term storage.

For thawing, the frozen capsule in silicone tube was transferred into pre-warmed GM in a 10-cm petri dish. After gentle agitation, the capsule was removed from the silicone tube and placed in 12-well culture plate containing GM.

As can be seen on **Figure 10**, GM-CSF secreting myoblast cells of the invention performed much better than control cell line (MVX1) after freezing glycerol (**Figures 10C**). The use of 10% glycerol (**Figures 10B and C**) as a freezing medium and an incubation time of 0 to 30 minute of the cells with the freezing medium before freezing was well tolerated.

### Example 4: Diversity of possible genetically modified immortalized human myoblast cell line to produce therapeutic proteins

The ability of immortalized myoblast cells according to the invention to be genetically engineered for the expression of complex molecules of therapeutic interest such as antibodies.

### Therapeutic macromolecules

### Myoblast transduction for the secretion of recombinant anti-human CD20 IgG (step ii))

For the expression of an anti-human CD20 IgG, the variable region of the rituximab heavy chain was inserted in a human IgG1 heavy chain backbone and the variable region of the rituximab light chain was inserted in a human kappa light chain backbone. The two sequences were synthetized and cloned and into a 3^{rd} generation pLV-hPGK-WPRE lentiviral vectors (Vectorbuilder). Cells were infected with the heavy and light chain vectors using same MOI for both transgenes as described in Lathuilière, 2016, Methods Mol Biol., 1448:139-155*.*
Immortalized myoblast cells obtained under Example 1 from clone 2 were transduced with lentiviral vectors encoding for the heavy and light chain of rituximab, a commercialized therapeutic anti-CD20 monoclonal antibody using methods already described (*Lathuiliere et al., 2016, supra*) with different dose of lentivirus (MOI) as described in Example 2. The secretion of IgG was then quantified in culture medium and the functionality of the secreted anti-CD20 IgG was demonstrated by flow cytometry. In a competition assay, human PBMC (peripheral blood mononuclear cell) were pre incubated with different concentration of either the myoblast-produced anti-CD20 IgG or commercial Rituximab and then incubated with a fluorescent tagged anti-CD20 antibody. Those experiments supported that the produced IgG behaved exactly like Rituximab in this assay as shown on **Figure 11A**. Moreover, the myoblast-produced IgG was as efficient in a degranulation assay that tested the induction of a cytotoxic response (overexpression of IFN-gamma or CD107a) after a B-cell line was exposed to either Rituximab or myoblast-produced anti-CD20 as shown on **Figure 11B and 11C**.

The obtained Anti-CD20 IgG secreting myoblasts were then loaded into an implantable device as described in Example 5 and under **Figure 9** (two cell populations tested CD20.30 & CD20.100, 10⁶ cells/device) and implanted in the mouse subcutaneous tissue. Before implantation, secretion of IgG from capsules is quantified in culture medium. Anti-CD20 IgG plasma level from living animals is then quantified every two weeks.

### Xenogenic proteins

In order to test the ability of the immortalized human myoblast cells of the invention to be genetically engineered to produce proteins from a different species, an immortalized myoblast clone obtained under Example 1 was transduced with different doses (MOI) of lentiviral vector encoding for the murine GM-CSF (mu-GM-CSF) protein. Individual mu-GM-CSF-secreting clones were then isolated from the transduced populations and one clone was selected and encapsulated into an implantable as described in Example 5 and under **Figure 9** (10⁶ cells/device). Delivery of muGM-CSF was quantified for several weeks *in vitro.*

The biological activity of the secreted muGM-CSF was confirmed by in vivo experiments in which a massive cellular immune response was detected around subcutaneously implanted encapsulation devices. The muGM-CSF secretion level was measured before implantation and after explantation at different timepoints (after 1, 3, 5 and 7 days). Some capsules were kept in vitro during the experiment as a comparator. A decrease in secretion level was observed, which is consistent with previous findings and the massive immune infiltrate produced by GM-CSF biological activity affects encapsulated cell survival. The influx of inflammatory cells may decrease oxygen and nutrient diffusion toward encapsulated cells.

All those data support that immortalized human myoblast cells of the invention present exceptional long term survival, while keeping myoblast features >100days, even in encapsulated environment. Those immortalized human myoblast cells can be genetically engineered to produce high level of protein of interest (such as as antibodies, antibody fragments, growth factors, cytokines etc..) in an encapsulated environment where they can proliferate. Finally those cells, once encapsulated, can be straightforwardly frozen after loading and thawed later on which is an important feature that will be very convenient for future clinical applications.

### Example 5: Comparing behavior of immortalized cells on the invention in different devices

In the following experiments, two types of implantable capsules are compared: a capsule as described in Fig. 9 for Groups A and B, vs a capsule as described in WO 2017/064571 (conventional capsule) for comparative Group C.
**Group A:** capsules are loaded with cells of the invention (immortalized human myoblast cell line expressing human GM-CSF (cell line deposited under number CCOS1901)
**Group B:** capsules are loaded with a control cell line: K562 human erythroleukemia cells expressing human GM-CSF
**Group C:** capsules are loaded with the same control cell line: K562 human erythroleukemia cells expressing human GM-CSF.
The conventional capsule does not enclose any supporting matrix, therefore it is not suited to be loaded with adherent cells such as human myoblast. To compare the efficacy of the two distinct capsules to produce huGM-CSF over time, the K562 human erythroleukemia cell line expressing human GM-CSF was used as an alternative cell line for producing the protein of interest. Group B and C provide a direct comparison of the performance of the two capsules as two distinct capsules contain the same genetically engineered cell line expressing the same therapeutic protein of interest according to the invention.
Under sterile culture condition, approximately 800'000 cells were loaded into the capsules. Capsules were maintained in culture medium at 37°C and 5% CO₂. The delivery of human GM-CSF was quantified in culture medium using an ELISA (Kit #KHC2011, Thermo Fischer) and reported in ng/24hrs (**Figure 12A**).

Capsules were then implanted in the mouse subcutaneous tissue for 1 week. After sacrifice, the capsules were retrieved from animals and placed in culture medium for the quantification of human GM-CSF delivery (**Figure 12B**). In addition, GM-CSF was quantified in the mouse serum (**Figure 12C**) as well as in the subcutaneous tissue surrounding the capsules (**Figure 12D**).

Those data support that the culture of known genetically engineered K562 human erythroleukemia cell line expressing human GM-CSF in a matrix containing macrocapsule as per an embodiment of the present invention represents already an improvement as compared to a capsule as described in WO 2017/064571. However, combination of the novel genetically engineered immortalized myoblast according to the invention in a matrix containing macrocapsule as per an embodiment of the present invention is by far the most efficient for achieving sustained, stable GM-CSF production both *in-vitro* and *in-vivo.*

## Claims

1. A method of establishing an immortalized human myoblast cell line, said method comprising the steps of:
a) Transducing at least one primary human myoblast cell with a lentiviral vector encoding for the cyclin-dependent kinase 4 (CDK4) gene and a lentiviral vector encoding for the catalytic subunit of the human telomerase (hTERT) gene to obtain immortalization of the said primary human myoblast cell;
b) Growing cells from at least one immortalized primary human myoblast cell obtained under step a) in a myoblast cell growth medium and isolating from the growth medium each obtained cell presenting at least one myoblast phenotypic marker in a separate culture medium;
c) Growing separately in individual culture and amplification media each isolated cell obtained under step b);
d) Selecting among all the individual culture and amplification media of step c) at least one cell line with improved stability or expression properties from the parental cell by single cell cloning;
e) Controlling the myogenic potential of the selected at least one cell line;
f) selecting individual clones based on their ability to survive in an encapsulation device;
g) Optionally repeating steps c) to f) sequentially to further improve the selected line.

2. A method according to claim 1, wherein step a) is carried out using two separate lentiviral vectors, such as of type pCLX or one single bicistronic vector.

3. An immortalized human myoblast cell line or a composition comprising immortalized human myoblast cells or a progeny thereof derived from primary human myoblast cells, wherein said cells express CDK4 and hTERT and retain myoblast features.

4. An immortalized myoblast cell line according to claim 3 presenting a triple positivity for CD56, CD82 and CD146.

5. An immortalized human myoblast cell line deposited with CCOS under accession number 1902 or a composition or a progeny thereof.

6. An immortalized human myoblast cell line or a composition comprising immortalized human myoblast cells or a progeny thereof derived from primary human myoblast cells obtainable from a method according to claim 1 or 2.

7. A method for the preparation of genetically engineered immortalized human myoblast cells expressing a therapeutic protein under hypoxic conditions, wherein said method comprises the steps of:
i) Providing at least one immortalized primary human myoblast cell;
ii) Transducing said at least one immortalized primary human myoblast cell with a lentiviral vector for the expression of a target protein under the control of a phosphoglycerate kinase (PGK) promoter;
iii) Growing separately in individual culture and amplification media each isolated cell obtained under step ii);
iv) Selecting among all the individual culture and amplification media of step iii) at least one cell line with the highest secretion level for the target protein;
v) Controlling the myogenic potential of the selected at least one cell line.

8. A method according to claim 7, wherein the target protein is human GM-CSF.

9. A genetically engineered immortalized human myoblast cell line or a composition comprising genetically engineered immortalized human myoblast cells or a progeny thereof, wherein said cells express CDK4, hTERT and therapeutic protein such as GM-CSF, retain myoblast features and are able to secrete said therapeutic protein.

10. A genetically immortalized human myoblast cell line secreting GM-CSF deposited with CCOS under accession number 1901 or a composition or a progeny thereof.

11. A genetically engineered immortalized human myoblast cell line or a composition or a progeny thereof obtainable from a method according to claims 7 or 8.

12. A genetically engineered immortalized human myoblast cells according to any one of claims 7 to 11 for use in encapsulated cell therapy.

13. A pharmaceutical composition comprising at least one immortalized human myoblast cell according to any one of claims 2 to 5 or 9 to 11 and a pharmaceutically acceptable carrier, diluent or excipient thereof.

14. A genetically engineered immortalized human myoblast cells according to any one of claims 9 to 11 for use in the prevention and/or treatment of a disorder or a disease, in particular a cancer, an inflammatory disorder or a neurodegenerative disorder.

15. An implantable biocompatible device or a kit comprising at least one immortalized human myoblast cell according to 9 to 19 in a cell culture medium, optionally further comprising one or more antigen(s).
